Europäisches Patentamt

European Patent Office

Office européen des brevets

(19)

(11) EP 0 752 247 B1

(12) **EUROPEAN PATENT SPECIFICATION**

(45) Date of publication and mention
of the grant of the patent:
**27.09.2000 Bulletin 2000/39**

(51) Int Cl.[7]: **A61K 31/57**

(21) Application number: **96304921.8**

(22) Date of filing: **03.07.1996**

(54) **Use of hydrocortisone or hydrocortisone salt and triclosan in the manufacture of a composition for the prevention and/or treatment of atopic eczema**

Verwendung von Hydrokortison oder Hydrokortisonsalz zur Herstellung einer Zusammensetzungen zur Verhütung und /oder Behandlung von atopischem Eczema

Utilisation d'hydrocortisone ou de sel d'hydrocortisone pour la fabrication d'une composition pour la prévention et/ou le traitement de l'eczema atopique

(84) Designated Contracting States:
**DE ES FR GB IT**

(30) Priority: **04.07.1995 GB 9513552**

(43) Date of publication of application:
**08.01.1997 Bulletin 1997/02**

(73) Proprietor: **SURTECH INTERNATIONAL LTD.**
**Moreton in Marsh GL56 0LH (GB)**

(72) Inventor: **Potts, Michael**
**Moreton in Marsh, Gloucester GL56 0LH (GB)**

(74) Representative: **Ritter, Stephen David et al**
**Mathys & Squire**
**100 Grays Inn Road**
**London WC1X 8AL (GB)**

(56) References cited:
DE-A- 2 125 893          US-A- 4 213 979
US-A- 4 512 987

- **CONTACT DERMATITIS, vol. 11, no. 3, 1984, pages 144-145, XP000602589 C.B.ARCHER ET AL.: "Chlorocresol sensitivity induced by treatment of allergic contact dermatitis with steroid creams"**
- **Z.HAUTKRANKH., vol. 54, no. 14, 1979, pages 668-670, XP000602593 P.KALLIOMÄKI ET AL.: "Eine vergleichende Untersuchung über die Wirksamkeit und Verträglichkeit der Crèmes CGP 4333 und GP41353 bei der Behandlung infektiéser Dermatiden"**
- **DRUG.THER.BULL., vol. 23, no. 21, 1985, pages 83-84, XP000602587 "Combining hydrocortisone with an antifungal on the skin"**
- **R.BERKOW ET AL.: "The Merck manual of Diagnosis and Therapy" 1992 , MERCK RESEARCH LABORATORIES , RAHWAY, N.J; USA XP002014216 * page 2405 ***

## Description

This invention relates to a composition for treating atopic eczema.

Atopic eczema (dermatitis) is a common chronic skin disorder, especially in children. The condition is characterized by inflammatory erythematous lesions which in mild cases are localized on the wrists, knees and neck but in severe cases a more widespread distribution is often noted.

Traditionally, atopic eczema has been treated with short term topical corticosteroids and maintained with emollients and bath oils. However atopic eczema is not curable at present and most available treatments involve the application of corticosteroids.

Hitherto, combinations of topical corticosteroids (in particular high potency steroids such as betamethasone-17-valerate), together with a powerful antibiotic (such as, for example, neomycin, or fusidic acid), have frequently been used to treat atopic eczema. The administration of the steroid was intended to combat the underlying physiological causes of the eczema as such, e.g. by promoting an antiinflammatory response, and the antibiotic was intended to counteract secondary infections. However, the most frequent sufferers of atopic eczema are children and the use of such strong combination therapies is undesirable. Also, topical neomycin and fucidic acid is known to cause hypersensitivity and reactions in the form of rashes and irritations may recur. Accordingly the need for other agents which do not suffer this disadvantage are required.

Recently, the presence of *Staphylococcus aureus* in eczematous lesions has been noted. In particular, high concentrations of *S. aureus* have been demonstrated in lesions of atopic eczema, even when they did not have the appearance of being infected. Further, the observed levels of *S. aureus* have been found to correlate with the severity of the eczema and it is now believed that the presence of *S. aureus* infections is implicated in the aetiology of the disease.

It has now been found that a topical composition comprising a low potency corticosteroid in combination with a chlorinated phenol antiseptic is especially effective in treating atopic eczema and does not suffer the disadvantages of known combination therapies. Further the use of a combination of a low potency corticosteroid and a chlorinated phenol antiseptic results in an enhanced activity in combating atopic eczema, compared to the use of a corticosteroid alone. Although chlorinated antiseptics have been used in topical compositions hitherto, their use in the treatment of atopic eczema, particularly in combination with low potency corticosteroid has never been suggested. In fact in view of their nonspecific action and chlorine-content, their use in connection with eczema would have tended to discourage such use.

When administered in combination with the chlorinated phenol antiseptic, the low potency corticosteroid has surprisingly been found to promote a sufficiently high antiinflammatory response to result in a significant reduction in the severity of the eczematous lesions, even to the extent of their disappearance. Further, a reduction in levels of *S. aureus* on and around the site of atopic eczema has been noted, but without total elimination of the normal skin flora. It is thus to be appreciated that the combination therapy according to the invention does not involve as a first effect, an anti-inflammatory action in response to the eczema plus an antibacterial effect on eczematous lesions that happened to be infected. Instead, the efficacy of the therapy of the invention seems to be based upon a synergistic effect whereby a low potency corticosteroid elicits a response (when administered in combination with a chlorinated phenol antiseptic) that hitherto was attainable only by using a high potency corticosteroid.

The preferred low potency corticosteroids used according to the invention are classified as having potencies "6" or "7" according to the United States Classification system wherein corticosteroids are classified on a scale of 1 to 7 ("1" being the most potent and "7" being less potent - See Goodman & Gilmans; The Pharmacological Directory of Therapeutics, 8th Edn.; McGraw-Hill Int. Ed.). In the corresponding United Kingdom Classification - see Mims (Monthly Index of Medical Specialities, Sept. 1995, Haymarket Publishing Services Ltd), - "low potency corticosteroids" correspond to those falling within class "7" on the United States scale. I.e. "7" on the US potency scale corresponds to "low potency" on the UK Mims classification).

Preferably, the low potency corticosteroid is present in a concentration of 0.1 to 5 % wt., most preferably 0.5 to 2.5 % wt. The chlorinated phenol antiseptic is preferably present in a concentration of 0.1 to 3 % wt., most preferably 0.5 to 1 % wt.

Advantageously in accordance with the invention there are provided compositions comprising a low potency corticosteroid and a chlorinated phenol antiseptic in the form of a solution, lotion, cream, ointment or gel suitable for topical application. Compositions according to the invention may contain any conventional incidental adjutants for example emollient, thickening agents, perfumes, colouring agents, stabilizing agents, pH adjusting agents and preservatives.

The preferred chlorinated phenol antiseptic used in accordance with the invention is triclosan, i.e. 5-chloro-2-(2,4-dichlorophenoxy)phenol, available e.g. under the Trade Name Irgasan DP300.

The preferred low potency corticosteroid is hydrocortisone or a hydrocortisone salt.

According to a further aspect of the invention there is provided the use of hydrocortisone or a hydrocortisone salt and triclosan in the manufacture of a composition for the prevention and/or treatment of atopic eczema. In carrying out the method of the invention, the compositions may be applied to affected region at regular intervals of, for example, 1-4 times a day.

The following example illustrates a composition according to the invention:

| HYDROCORTISONE/TRICLOSAN CREAM | | |
|---|---|---|
| A. | Cetomacrogol 1000 BP | 1.000 |
| | Mineral Oil | 30.000 |
| | Lasemul 92AE | 3.500 |
| | Cetyl Alcohol Pure 1692 | 3.500 |
| | Cutina CP | 2.000 |
| | Lasemul 130 | 3.000 |
| | Silicone DC200/350 | 2.000 |
| | Irgasan DP300 (triclosan) | 0.700 |
| B. | Water - deionised | 46.834 |
| | Glycerine | 3.000 |
| | EDTA Disodium | 0.100 |
| C. | Water - deionised | 3.000 |
| | Chlorhexadine Digluconate | 0.250 |
| | Hydrocortisone Acetate BP | 1.116 |
| | | 100.000 |

## Claims

1. The use of (i) hydrocortisone or a hydrocortisone salt and (ii) triclosan in the manufacture of a composition for the prevention and/or treatment of atopic eczema.

2. The use according to Claim 1 wherein the hydrocortisone or hydrocortisone salt is present in a concentration of 0.1 to 5 % wt.

3. The use according to Claim 2 wherein the hydrocortisone or hydrocortisone salt is present in a concentration of 0.5 to 2.5 % wt.

4. The use according to any preceding claim wherein the triclosan is present in a concentration of 0.1 to 3 % wt.

5. The use according to Claim 4 wherein the triclosan is present in a concentration of 0.5 to 1 % wt.

6. The use according to any preceding claim in the composition is in the form of a solution, lotion, cream, ointment or gel.

## Patentansprüche

1. Verwendung von (i) Hydrocortison oder eines Hydrocortisonsalzes und (ii) Triclosan bei der Herstellung einer Zubereitung für die Prophylaxe und/oder Behandlung atopischer Ekzeme.

2. Verwendung nach Anspruch 1, bei der das Hydrocortison oder Hydrocortisonsalz in einer Konzentration von 0,1 bis 5 Gew.-% vorliegt.

3. Verwendung nach Anspruch 2, bei der das Hydrocortison oder Hydrocortisonsalz in einer Konzentration von 0,5 bis 2,5 Gew.-% vorliegt.

4. Verwendung nach einem der vorstehenden Ansprüche, bei der das Triclosan in einer Konzentration von 0,1 bis 3 Gew.-% vorliegt.

5. Verwendung nach Anspruch 4, bei der das Triclosan in einer Konzentration von 0,5 bis 1 Gew.-% vorliegt.

6. Verwendung nach einem der vorstehenden Ansprüche, worin die Zubereitung in Form einer Lösung, Lotion, Creme, Salbe oder Gel vorliegt.

## Revendications

1. Utilisation (i) d'hydrocortisone ou d'un sel d'hydrocortisone et (ii) de triclosan dans la fabrication d'une composition pour la prévention et/ou le traitement de l'eczéma atopique.

2. Utilisation selon la revendication 1, selon laquelle l'hydrocortisone ou le sel d'hydrocortisone est présent à une concentration de 0,1 à 5 % en poids.

3. Utilisation selon la revendication 2, selon laquelle l'hydrocortisone ou le sel d'hydrocortisone est présent à une concentration de 0,5 à 2,5 % en poids.

4. Utilisation selon l'une quelconque des revendications précédentes, selon laquelle le triclosan est présent à une concentration de 0,1 à 3 % en poids.

5. Utilisation selon la revendication 4, selon laquelle le triclosan est présent à une concentration de 0,5 à 1 % en poids.

6. Utilisation selon l'une quelconque des revendications précédentes, selon laquelle la composition est sous la forme d'une solution, d'une lotion, d'une crème, d'une pommade ou d'un gel.